Europäisches Patentamt

European Patent Office

Office européen des brevets

(19)

(11) Veröffentlichungsnummer: **0 280 887 B1**

(12) **EUROPÄISCHE PATENTSCHRIFT**

(45) Veröffentlichungstag der Patentschrift: **08.01.92**

(51) Int. Cl.5: **A61K 9/10**

(21) Anmeldenummer: **88101411.2**

(22) Anmeldetag: **01.02.88**

(54) **Mischmicell-Lösungen mit nichtsteroidalen Entzündungshemmern.**

(30) Priorität: **03.02.87 CH 380/87**

(43) Veröffentlichungstag der Anmeldung:
**07.09.88 Patentblatt 88/36**

(45) Bekanntmachung des Hinweises auf die
Patenterteilung:
**08.01.92 Patentblatt 92/02**

(84) Benannte Vertragsstaaten:
**AT BE CH DE ES FR GB IT LI LU NL SE**

(56) Entgegenhaltungen:
**EP-A- 0 167 825       DE-A- 2 730 570**
**DE-A- 3 221 579       GB-A- 2 046 094**
**US-A- 3 197 368       US-A- 3 896 145**

**JOURNAL OF PHARMACEUTICAL SCIENCES,
Band 55, Nr. 9, September 1966, Seiten
901-906, American Pharmaceutical Association, Easton, PA, US; T.R. BATES et al.:
"Solubilizing properties of bile salt solutions
II"**

(73) Patentinhaber: **F. HOFFMANN-LA ROCHE AG**
**Postfach 3255**
**CH-4002 Basel(CH)**

(72) Erfinder: **Ferro, Alberto, Dr.**
**Gstaltenrainweg 67**
**CH-4125 Riehen(CH)**
Erfinder: **Steffen, Hans, Dr.**
**Seestrasse 16**
**CH-4410 Liestal(CH)**

(74) Vertreter: **Lederer, Franz, Dr. et al**
**Patentanwalt Dr. Franz Lederer Lucile-
Grahn-Strasse 22**
**W-8000 München 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Bei der parenteralen Verabreichung von nicht-steroidalen Entzündungshemmern werden häufig lokale Irritationen und hämolytische Effekte beobachtet. Der vorliegenden Erfindung liegt die Aufgabe zugrunde, eine besser verträgliche parenterale Applikationsform für nicht-steroidale Entzündungshemmer verfügbar zu machen.

Aus der deutschen Offenlegungsschrift 2 730 570 ist bekannt, Mischmicellen aus Gallensäuren und Lipiden zur Solubilisierung schwer oder nicht wasserlöslicher pharmazeutischer Wirkstoffe in wässrigem Medium zu verwenden. Ueberraschend wurde gefunden, dass wässrige Mischmicell-Lösungen von nicht-steroidalen Entzündungshemmern bei parenteraler Verabreichung wesentlich besser verträglich sind als wässrig-organische oder selbst rein wässrige Lösungen solcher Entzündungshemmer, die nicht unter Verwendung von Mischmicellen hergestellt wurden.

Die vorliegende Erfindung betrifft somit wässrige Mischmicell-Lösungen, enthaltend ein Salz einer Gallensäure ein Lipid, und einen nicht-steroidalen Entzündungshemmer.

In anderer Hinsicht betrifft die Erfindung die Verwendung von Mischmicellen aus Gallensäuresalzen und Lipiden zur Solubilisierung von nicht-steroidalen Entzündungshemmern in wässrigem Milieu.

Als Gallensäuresalze kommen in den vorliegenden Mischmicell Lösungen die in der DE-OS 2 730 570 erwähnten Salze von Gallensäuren bzw. Gallensäurederivaten in Betracht, insbesondere Cholate, Glycocholate und Taurocholate, insbesondere die Alkalisalze, wie die Natriumsalze. Besonders bevorzugt ist Na-Glycocholat.

Als Lipide kommen insbesondere Phosphatidylcholine, z.B. natürliche Lecithine oder synthetische Lecithine mit abgewandelten Seitenketten (z.B. solche, die in der europäischen Patentanmeldung A2-0154977 beschrieben sind) in Betracht. Bevorzugt sind natürliche Lecithine wie Ei- oder Soja-Lecithin.

Nicht-steroidale Entzündungshemmer (non-steroidal anti-inflammatory drugs, NSAID's) im Sinne der vorliegenden Erfindung sind Verbindungen, die sich von Steroiden strukturell unterscheiden und eine entzündungshemmende Wirkung entfalten. Häufig sind solche Verbindungen durch die Anwesenheit einer Carbonsäuregruppe charakterisiert und/oder sind Derivate der Essigsäure oder der Propionsäure. Beispiele solcher nicht-steroidalen Entzündungshemmer sind Carprofen, Ibuprofen, Benoxaprofen, Naproxen, Sulindac, Zomepirac, Fenclofenac, Alclofenac, Ibufenac, Flunixin, Indomethacin oder Salze davon. Ein im Rahmen der vorliegenden Erfindung bevorzugter nicht-steroidaler Entzündungshemmer ist Carprofen (6-Chlor-α-methyl-carbazol-2-essigsäure) und physiologisch verträgliche Salze davon mit Basen, z.B. Alkalimetallhydroxiden, Aminen oder basischen Aminosäuren wie Arginin oder Lysin.

Das Molverhältnis zwischem Lipid und der Gallensäure liegt zweckmässig in der Grössenordnung von 0,1:1 bis 2:1. Bevorzugt sind Mischungsverhältnisse von 0,8:1 bis 1,5:1.

Der Anteil von Lipid plus Gallensäure in der Injektionslösung kann über weite Grenzen variieren und z.B. 50-300 mg/ml Injektionslösung betragen.

Der Anteil des Pharmakons in den erfindungsgemässen Lösungen kann ebenfalls über weite Grenzen variieren und z.B. 0,1-100 mg/ml Lösung betragen. Mittels der erfindungsgemässen Lösungen können relativ grosse Wirkstoffmengen in einer Volumeneinheit Vehikel solubilisiert werden, was besonders bei der Behandlung von Grosstieren von Vorteil ist.

Die erfindungsgemässen Mischmicell-Lösungen können durch blosses Vermischen der einzelnen Bestandteile hergestellt werden. In einer anderen Ausführungsform kann man das Lipid, die Gallensäure und eine zur Salzbildung damit geeignete Base, z.B. ein Alkalihydroxid, oder direkt das Gallensäuresalz sowie den Wirkstoff in einem organischen Lösungsmittel lösen, darauf das organische Lösungsmittel abdampfen und hierauf Wasser, ggf. isotonisierende Zusätze und gegebenenfalls weitere Ingredientien zugeben, wobei in der Regel die isotonisierenden Zusätze und zumeist auch die allfälligen weiteren Ingredientien vor der Zugabe zum erwähnten Eindampfrückstand mit dem Wasser vermischt werden. Als organische Lösungsmittel kommen solche in Betracht, in denen die zu lösenden Komponenten hinreichend löslich sind, wie z.B. niedere Alkanole, insbesondere Methanol oder Aethanol.

In einer weiteren Ausführungsform kann man zunächst eine wässrige Mischmicell-Lösung aus einem Lipid und einem Gallensäuresalz herstellen und sodann den Wirkstoff zugeben.

Die Zeit, die benötigt wird, bis das so erhaltene Gemisch nach Rühren homogen erscheint, hängt von der Art der Gallensäure, des Lipids, des Wirkstoffs und deren Konzentrationen ab und kann in der Regel durch kurzzeitiges Erwärmen verkürzt werden.

Zweckmässig werden die erfindungsgemässen Mischmicell-Lösungen auf einen pH-Wert von etwa 5,5-7,5 eingestellt.

Die erfindungsgemässen Mischmicell-Lösungen können zusätzlich Hilfsstoffe enthalten, z.B. Puffer, isotonisierende Zusätze, Stabilisatoren und/oder Konservierungsmittel, z.B. Benzylalkohol. Als isotonisierende Zusätze kommen insbesondere in Betracht: Natriumchlorid, Mannit oder Glucose. Als Puffer können Tris-Puffer, Phosphat-Puffer, Citrat-Puffer, Citrat-Phosphat-Mischpuffer, usw. verwendet werden. Der osmotische Druck der erfindungsgemässen Injektionslösungen sollte im Idealfall demjenigen des Blutes entsprechen, d.h. etwa 300 mOsm betragen, kann aber in gewissen Grenzen variieren.

Weiterhin kann es zweckmässig sein, die Herstellung der erfindungsgemässen Lösungen unter Inertgas vorzunehmen und der Lösung ein Antioxidans, wie z.B. Natriumascorbat, Natriumhydrogensulfit oder Natriumpyrosulfit zuzusetzen.

Eine bevorzugte erfindungsgemässe Mischmicell-Lösung enthält Natriumglycocholat, ein natürliches Lecithin und Carprofen oder ein Salz davon, insbesondere das Arginin- oder Lysinsalz.

Eine solche Lösung eignet sich insbesondere zur Anwendung in der Veterinärmedizin, z.B. zur Behandlung akuter und chronischer Laminitis: Skeletterkrankungen wie Navicular-Krankheit; Myositis: Schmerzen bei Koliken, besonders Flatulenz und spastischer Kolik; Schmerzen bei Erkrankungen der Atemwege; akuter Mastitis: traumatischen Schmerzen und zur Behandlung von Erkrankungen in der Puerperalphase, wie Mastitis, fehlender oder ungenügender Uterusinvolution (postpartiale Involution).

Die nachfolgenden Beispiele erläutern die Erfindung weiter.

Beispiel 1

a) 8,85 g Glycocholsäure werden in 50 ml $N_2$-begastem Wasser zu Injektionszwecken suspendiert und mit Hilfe von 1,9 ml frisch hergestellter NaOH 40% aufgelöst.

b) 16,9 g fein zerschnittenes Lecithin werden hinzugefügt und unter gutem Rühren gelöst.

c) Die erhaltene Mischmicell-Lösung wird auf ca. 50-60°C erwärmt.

d) 3 g L-Arginin werden bei ca. 40°C in 15 ml $N_2$-begastem Wasser zu Injektionszwecken aufgelöst.

e) 5 g Carprofen-Substanz werden in die bei ca, 50-60° vorerwärmte Mischmicell-Lösung c) suspendiert und unter portionenweiser Zugabe der L-Arginin-Lösung d) aufgelöst.

f) Die erhaltene Lösung wird mit 2N HCl auf pH 6,0±0,2 eingestellt und mit $N_2$-begastem Wasser zu Injektionszwecken auf das Endvolumen von 100 ml ergänzt.

g) Die Lösung wird durch ein Membranfilter à 0,45 $\mu$m filtriert, unter aseptischen Bedingungen und $N_2$-Atmosphäre in Ampullen abgefüllt und im Autoklaven sterilisiert.

Beispiel 2

Man verfährt wie in Beispiel 1, setzt jedoch nach der Operation e)1,5g Benzylalkohol zu.

Beispiel 3

9,86 g Glycocholsäure (mit 5,6% $H_2O$) und 3,48 g L-Arginin werden in 60 ml Aethanol-Methanol (2:1) bei ca. 40°C gelöst. 17,77 g Lecithin werden zugefügt und gelöst. Im Rotationsverdampfer wird unter Vakuum das organische Lösungsmittel verdampft, wobei sich ein Schaum bildet. 4,0 g Indomethacin und 1,95 g L-Arginin werden in 55 ml $N_2$-begastem Wasser bei Raumtemperatur gelöst. Die erhaltene Lösung wird zu dem wie vorstehend beschrieben erhaltenen Schaum gegeben und die Masse unter Rühren gelöst. Die erhal- tene Mischmicell-Lösung wird mit 2N HCl auf pH 7,1 ± 0,1 eingestellt und mit $N_2$-begastem Wasser zu Injektions- zwecken auf das Endvolumen von 100 ml ergänzt. Danach wird wie in Beispiel 1g) verfahren.

Beispiel 4

9,86 g Glycocholsäure (mit 5,6% $H_2O$) und 3,48 g L-Arginin werden in 50 ml $N_2$-begastem Wasser zu Injektionszwecken aufgelöst. Danach wird wie in Beispiel 1b) und c) verfahren, wobei jedoch 17,77 g Lecithin eingesetzt werden. 2,5 g Ibuprofen und 2,11 g L-Arginin werden in 20 ml Methanol gelöst, worauf das Methanol im Rotationsverdampfer eliminiert wird. Das erhaltene Pulver wird in die Mischmicell-Lösung gegeben und unter Rühren aufgelöst. Danach wird mit 2N HCl auf pH 7,1 ± 0,1 eingestellt, mit $N_2$-begastem Wasser für Injektionszwecke auf 100 ml aufgefüllt und wie in Beispiel 1g) verfahren.

Beispiel 5

9.86 g Glycocholsäure (mit 5,6% $H_2O$) und 3.48 g L-Arginin werden in 50 ml $N_2$-begastem Wasser zu Injektionszwecken aufgelöst. 2,5 g Naproxen und 1,89 g L-Arginin werden der erhaltenen Lösung zugefügt und unter Rühren gelöst. 17.77 g fein zerschnittenes Lecithin werden hinzugefügt und unter Rühren bei ca. 40-50°C gelöst. Danach wird wie in Beispiel 3 das pH eingestellt, die Lösung auf 100 ml ergänzt, filtriert und in Ampullen abgefüllt.

Beispiel 6

Zur Prüfung der lokalen Verträglichkeit wurden die Präparate A (Kontrollwert), B (erfindungsgemässe Formulierung) C und D (konventionelle Formulierungen) einmal täglich während 14 Tagen intravenös an Hunde verabreicht.

A:    NaCl 0.9%-ig

B:    Carprofen                 -

50,0 mg

L-Arginin -

30,0 mg

Glycocholsäure (wasserfrei) 88,5 mg

NaOH

40% 1-

9,0 μl

Lezithin für Mischmizellen 169,0 mg

HCl (2N, pH 6,0)-

q.s.

Wasser zu Injektionszwecken ad 1,0 ml

C: Carprofen -

10,0 mg

Polyäthylenglykol

400 600,0 μl

Wasser zu Injektionszwecken

ad 1,0 ml

D: Carprofen -

25,0 mg

Diäthanolamin -

19,0 mg

EDTA-Dinatriumsalz 0,1 mg

Benzylalkohol -

10,0 μl

Wasser zu Injektionszwecken

ad 1,0 ml

Die medikierten Tiere erhielten 20 mg/kg Wirkstoff. Die Injektionen wurden jeweils an der gleichen Stelle (V.cephalica antebrachii) vorgenommen.

Resultate:

Bei dem mit C behandelten Tiere trat nach 3 Injektionen eine Entzündung an der Applikationsstelle auf und die Vene war nach 6 Injektionen verschlossen.

Bei dem mit D behandelten Tier entzündete sich die Applikationsstelle bereits nach der ersten Verabreichung. Eine dritte Verabreichung war infolge einer Venen-Obliteration nicht möglich. Bei dem mit B behandelten Tier und bei dem Kontrolltier (A) zeigten sich nach 14 Injektionen keine Veränderungen an der Applikationsstelle. Bei der Sektion zeigten sich bei den mit C und D behandelten Tieren Thrombophlebitiden an der Injektionsstelle; das mit B behandelte Tier war ohne Befund.

**Patentansprüche**

**Patentansprüche für folgende Vertragsstaaten : AT, BE, CH, DE, FR, GB, IT, LU, NL, SE**

1. Wässrige Mischmicell-Lösungen, enthaltend ein Salz einer Gallensäure, ein Lipid, und einen nicht-steroidalen Entzündungshemmer.

2. Mischmicell-Lösung gemäss Anspruch 1, in der die Gallensäure Cholsäure, Glycocholsäure oder Taurocholsäure ist.

3. Mischmicell-Lösung gemäss Anspruch 1 oder 2, in der das Lipid ein Phosphatidylcholin, insbesondere ein natürliches Lecithin ist.

4. Mischmicell-Lösung gemäss den Ansprüchen 1-3, in der der nicht-steroidale Entzündungshemmer ein Carbonsäurederivat ist.

5. Mischmicell-Lösung gemäss Anspruch 4, in der der nicht-steroidale Entzündungshemmer ein Essigsäure- oder Propionsäurederivat ist.

6. Mischmicell-Lösung gemäss Anspruch 4, in der der nicht-steroidale Entzündungshemmer Carprofen, Ibuprofen, Benoxaprofen, Naproxen, Sulindac, Zomepirac, Fenclofenac, Alclofenac, Ibufenac, Flunixin, Indomethacin oder ein Salz davon ist.

7. Mischmicell-Lösung gemäss den Ansprüchen 1-5, in der der nicht-steroidale Entzündungshemmer Carprofen ist.

8. Mischmicell-Lösung gemäss Anspruch 1, enthaltend Na-Glycocholat, ein natürliches Lecithin und Carprofen.

9. Verfahren zur Herstellung von Mischmicell-Lösungen von Anspruch 1, dadurch gekennzeichnet, dass man die Bestandteile miteinander vermischt.

10. Verwendung von Mischmicellen aus Gallensäuresalzen und Lipiden zur Herstellung lokal verträglicher, wässriger Injektionslösungen von nicht-steroidalen Entzündungshemmern.

**Patentansprüche für folgenden Vertragsstaat : ES**

1. Verfahren zur Herstellung wässriger Mischmicell-Lösungen, enthaltend ein Salz einer Gallensäure, ein Lipid, und einen nicht-steroidalen Entzündungshemmer, dadurch gekennzeichnet, dass man die Bestandteile miteinander vermischt.

2. Verfahren gemäss Anspruch 1, worin die Gallensäure Cholsäure, Glycocholsäure oder Taurocholsäure ist.

3. Verfahren gemäss Anspruch 1 oder 2, worin das Lipid ein Phosphatidylcholin, insbesondere ein natürliches Lecithin ist.

4. Verfahren gemäss den Ansprüchen 1-3, worin der nicht-steroidale Entzündungshemmer ein Carbonsäurederivat ist.

5. Verfahren gemäss Anspruch 4, worin der nicht-steroidale Entzündungshemmer ein Essigsäure- oder Propionsäurederivat ist.

6. Verfahren gemäss Anspruch 4, worin der nicht-steroidale Entzündungshemmer Carprofen, Ibuprofen, Benoxaprofen, Naproxen, Sulindac, Zomepirac, Fenclofenac, Alclofenac, Ibufenac, Flunixin, Indomethacin oder ein Salz davon ist.

7. Verfahren gemäss den Ansprüchen 1-5, worin der nicht-steroidale Entzündungshemmer Carprofen ist.

8. Verwendung von Mischmicellen aus Gallensäuresalzen und Lipiden zur Herstellung lokal verträglicher, wässriger Injektionslösungen von nicht-steroidalen Entzündungshemmern.

## Claims
## Claims for the following Contracting States : AT, BE, CH, DE, FR, GB, IT, LU, NL, SE

1. Aqueous mixed micelle solutions containing a salt of a cholanic acid, a lipid and a non-steroidal anti-inflammatory.

2. A mixed micelle solution in accordance with claim 1, in which the cholanic acid is cholic acid, glycocholic acid or taurocholic acid.

3. A mixed micelle solution in accordance with claim 1 or 2, in which the lipid is a phosphatidylcholine, especially a natural lecithin.

4. A mixed micelle solution in accordance with claims 1-3, in which the non-steroidal anti-inflammatory is a carboxylic acid derivative.

5. A mixed micelle solution in accordance with claim 4, in which the non-steroidal anti-inflammatory is an acetic acid or propionic acid derivative.

6. A mixed micelle solution in accordance with claim 4, in which the non-steroidal anti-inflammatory is carprofen, ibuprofen, benoxaprofen, naproxen, sulindac, zomepirac, fenclofenac, alclofenac, ibufenac, flunixin, indomethacin or a salt thereof.

7. A mixed micelle solution in accordance with claims 1-5, in which the non-steroidal anti-inflammatory is carprofen.

8. A mixed micelle solution in accordance with claim 1, containing Na glycocholate, a natural lecithin and carprofen.

9. A process for the manufacture of the mixed micelle solutions of claim 1, characterized by mixing the ingredients with one another.

10. The use of mixed micelles from cholanic acid salts and lipids for the manufacture of locally tolerable aqueous injection solutions of non-steroidal anti-inflammatories.

## Claims for the following Contracting State : ES

1. A process for the manufacture of aqueous mixed micelle solutions containing a salt of a cholanic acid, a lipid and a non-steroidal anti-inflammatory, characterized by mixing the ingredients with one another.

2. A process in accordance with claim 1, wherein the cholanic acid is cholic acid, glycocholic acid or taurocholic acid.

3. A process in accordance with claim 1 or 2, wherein the lipid is a phosphatidylcholine, especially a natural lecithin.

4. A process in accordance with claims 1-3, wherein the non-steroidal anti-inflammatory is a carboxylic acid derivative.

5. A process in accordance with claim 4, wherein the non-steroidal anti-inflammatory is an acetic acid or propionic acid derivative.

6. A process in accordance with claim 4, wherein the non-steroidal anti-inflammatory is carprofen, ibuprofen, benoxaprofen, naproxen, sulindac, zomepirac, fenclofenac, alclofenac, ibufenac, flunixin, indomethacin or a salt thereof.

7. A process in accordance with claims 1-5, wherein the non-steroidal anti-inflammatory is carprofen.

8. The use of mixed micelles from cholanic acid salts and lipids for the manufacture of locally tolerable aqueous injection solutions of non-steroidal anti-inflammatories.

**Revendications**
**Revendications pour les Etats contractants suivants : AT, BE, CH, DE, FR, GB, IT, LU, NL, SE**

1. Solutions aqueuses micellaires mélangées contenant un sel d'un acide biliaire, un lipide et un agent anti-inflammatoire non stéroïdique.

2. Solution micellaire mélangée selon la revendication 1, dans laquelle l'acide biliaire est l'acide cholique, l'acide glycocholique ou l'acide taurocholique.

3. Solution micellaire mélangée selon revendication 1 ou 2, dans laquelle le lipide est une phosphatidylcholine, en particulier une lécithine naturelle.

4. Solution micellaire mélangée selon revendications 1 à 3, dans laquelle l'agent anti-inflammatoire non stéroïdique est un dérivé d'acide carboxylique.

5. Solution micellaire mélangée selon revendication 4, dans laquelle l'agent anti-inflammatoire non stéroïdique est un dérivé de l'acide acétique ou de l'acide propionique.

6. Solution micellaire mélangée selon revendication 4, dans laquelle l'agent anti-inflammatoire non stéroïdique est le Carprofen, l'Ibuprofen, le Benoxaprofen, le Naproxen, le Sulindac, le Zomepirac, le Fenclofenac, l'Alclofenac, l'Ibufenac, le Flunixin, l'Indomethacin ou l'un de leurs sels.

7. Sclution micellaire mélangée selon revendications 1 à 5, dans laquelle l'agent anti-inflammatoire non stéroïdique est le Carprofen.

8. Solution micellaire mélangée selon revendication 1, contenant du glycocholate de sodium, une lécithine naturelle et du Carprofen.

9. Procédé de préparation des solutions micellaires mélangées de la revendication 1, caractérisé en ce que l'on mélange les composants entre eux.

10. Utilisation de micelles mélangées de sels d'acide biliaire et de lipides pour la préparation de solutions aqueuses pour injections d'agents anti-inflammatoires non stéroïdiques bien tolérées localement.

**Revendications pour l'Etat contractant suivant : ES**

1. Procédé de préparation de solutions aqueuses micellaires mélangées contenant un sel d'un acide biliaire, un lipide et un agent anti-inflammatoire non stéroïdique, caractérisé en ce que l'on mélange les composants entre eux.

2. Procédé selon la revendication 1, dans lequel l'acide biliaire est l'acide cholique, l'acide glycocholique ou l'acide taurocholique.

3. Procédé selon la revendication 1 ou 2, dans lequel le lipide est une phosphatidylcholine, en particulier une lécithine naturelle.

4. Procédé selon les revendications 1 à 3, dans lequel l'agent anti-inflammatoire non stéroïdique est un dérivé d'acide carboxylique.

5. Procédé selon la revendication 4, dans lequel l'agent anti-inflammatoire non stéroïdique est un dérivé de l'acide acétique ou de l'acide propionique.

6. Procédé selon la revendication 4, dans lequel l'agent anti-inflammatoire non stéroïdique est le Carprofen, l'Ibuprofen, le Benoxaprofen, le Naproxen, le Sulindac, le Zomepirac, le Fenclofenac, l'Alclofenac, l'Ibufenac, le Flunixin, l'Indomethacin ou l'un de leurs sels.

7. Procédé selon les revendications 1 à 5, dans lequel l'agent anti-inflammatoire non stéroïdique est le Carprofen.

8. Utilisation de micelles mélangées de sels d'acide biliaire et de lipides pour la préparation de solutions aqueuses pour injections d'agents anti-inflammatoires non stéroïdiques bien tolérées localement.